# EUROPEAN PATENT SPECIFICATION

(11) **EP 1 858 596 B1**
(45) Date of publication and mention of the grant of the patent: **01.12.2010**
(21) Application number: 06824677.6
(22) Date of filing: 01.03.2006
(51) Int. Cl.: A61Q 15/00, A61K 8/26

(54) **ANHYDROUS ANTIPERSPIRANT COMPOSITION CONTAINING SKIN SOFTENERS**
WASSERFREIE, SCHWEISSHEMMENDE ZUSAMMENSETZUNG MIT HAUTSCHONENDEN ELEMENTEN
COMPOSITION ANHYDRE ANTITRANSPIRATION CONTENANT DES LOTIONS ADOUCISSANTES

(30) Priority: 17.03.2005 US 82295
(43) Date of publication of application: 28.11.2007
(73) Proprietor: The Gillette Company, Boston, MA 02199 (US)
(72) Inventor: GALANTE, Cheryl, Lynn, Marshfield, MA 02050 (US); ELLIOTT, David, L., Pineville, LA 71360-5026 (US)
(74) Representative: Wilding, Richard Alan
(86) International application number: PCT/US2006/007488
(87) International publication number: WO 2007/030139

(56) References cited:
- US-A- 5 833 382
- US-A- 6 051 216
- US-B1- 6 353 076

## Description

The present invention relates to anhydrous antiperspirant compositions comprising skin and/or hair softeners.

It is important in the formulation of antiperspirant compositions for women that such compositions have desirable aesthetic attributes as well as excellent antiperspirant efficacy. In particular, it is highly desirable to formulate such products to have improved skin feel (i.e. soft skin), good moisturization, and softer, less noticeable hair.

US 5 833 382 discloses an anhydrous solid antiperspirant composition containing antiperspirant actives, behenoxy dimethicone, a non-volatile oil and volatile silicone in a dispensing container.

It has now been discovered that antiperspirant compositions with improved skin and hair feel can be made by including behenoxy dimethicone and a non-polar, non-volatile liquid emollient oil.

The present invention embraces an anhydrous topical antiperspirant composition comprising a dermatologically acceptable carrier and a perspiration reducing effective amount of a particulate antiperspirant salt suspended in the carrier. In addition, the composition will include behenoxy dimethicone, a non-polar, non-volatile liquid emollient oil and a surfactant. Such a composition provides improved skin feel (i.e. softer skin), good moisturization and softer, less noticeable hair (or hair stubble). The present invention also embraces a method of reducing perspiration from human skin by applying the aforementioned antiperspirant composition.

The anhydrous topical antiperspirant composition of the present invention comprises a dermatologically acceptable anhydrous carrier, a perspiration reducing effective amount of a particulate antiperspirant salt suspended in the carrier, behenoxy dimethicone, a non-polar, non-volatile liquid emollient oil and a surfactant. The term "anhydrous" as used herein means that the composition is substantially free (that is, contains less than about 2%, preferably less than 1%, and most preferably less than 0.1 % by weight) of free water (excluding any water of hydration associated with the antiperspirant salt or other components of the composition).

The particulate antiperspirant salt may be selected from any of the known aluminum and aluminum-zirconium antiperspirant salts, particularly the enhanced efficacy antiperspirant salts. Preferred salts are the aluminum-zirconium chlorohydrates, including the tri-, tetra-, penta- and octa-chlorohydrate forms, with aluminum-zirconium pentachlorohydrate being most preferred. Most preferred are the enhanced efficacy forms of such salts, particularly those salts with HPLC peak 4 to peak 3 area ratios greater than 0.5, as well as those salts with HPLC peak 5 area greater than 30%.

The particulate antiperspirant salt is suspended in an anhydrous, dermatologically acceptable carrier, particularly a carrier comprising a silicone (e.g., cyclomethicone, dimethicone, etc.), typically at a concentration of about 3% to about 25% (USP) active by weight, more typically at about 6% to about 22% (USP) active by weight.

The antiperspirant composition of the present invention also includes, behenoxy dimethicone, preferably at a concentration of about 0.3% to about 3% by weight of the composition, more preferably at about 0. 5% to about 2.5% by weight, most preferably at about 1% to about 2% by weight.

The antiperspirant composition further includes a non-polar, non-volatile liquid emollient oil. The emollient oil may be selected from any of those emollient oils currently known for use in antiperspirant compositions, such as, for example, those emollient oils described in U.S. 5,733,534. The emollient oil preferably may be included in an amount of about 3% to about 30% by weight of the composition, more preferably about 5% to about 25% by weight, most preferably about 10% to about 20% by weight. Examples of typical emollient oils include silicone oils, hydrocarbon oils, esters of mono and dibasic carboxylic acids with mono- and polyhydric alcohols, esters of fatty alcohols and benzoic acid, polyethoxylated and/or polypropoxylated ethers of fatty alcohols, etc., and mixtures of such emollient oils. A preferred emollient oil is a fatty alkyl benzoate, such as C₁₂₋₁₅ alcohols benzoate (C₁₂₋₁₅ alkyl benzoate).

The antiperspirant composition includes a surfactant, preferably in an amount of about 0.5% to about 5% by weight, more preferably in an amount of about 1% to about 3% by weight. It has been found that the surfactant improves the antiperspirant efficacy of the composition. This is particularly desirable with the present compositions because the behenoxy dimethicone and emollient oil can diminish antiperspirant efficacy. The inclusion of surfactant in the composition appears to prevent any diminishment in overall antiperspirant efficacy. A preferred surfactant will have an HLB greater than 5, typically 9 to 16. A particularly preferred surfactant is C₂₀₋₄₀ Pareth-10.

The antiperspirant composition further includes a dermatologically acceptable anhydrous carrier. The anhydrous carrier may comprise any of the ingredients commonly utilized in the formulation of topical antiperspirant compositions. Advantageously, the carrier vehicle will comprise one or more volatile silicones, which evaporate quickly and provide a dry feel. The volatile silicones include the cyclic polydimethylsiloxanes, also known as cyclomethicones, which have from about three to about seven silicon atoms, and the linear polydimethylsiloxanes, also known as dimethicones, which have from about 2 to about 8 silicon atoms. The linear volatile silicones generally have viscosities of less than 5 cst, while the cyclic volatile silicones have viscosities under 10 cst. Mixtures of volatile silicones may be advantageously employed. When included in the carrier, the volatile silicones are typically present in an amount of about 10% to 90%, more typically about 20% to 60%, by weight.

The carrier may also optionally include waxes such as fatty alcohols, for example, stearyl alcohol, cetyl alcohol, and myristyl alcohol, fatty amides, for example, Stearamide MEA and Lauramide DEA, hydrogenated castor oil (castor wax) and polyethylene homopolymer, gelling agents such as 12-hydroxystearic acid (including esters and amides thereof) and glyceryl tribehenate, N-acyl amino acid amides such as N lauroyl-L-glutamic acid-di-n-butyl amide and alkyl amides such as 2-dodecyl-N,N'-dibutylsuccinamide, suspending agents such as clays (e.g. quaternium-18 hectorite) and silicas, and fillers such as talc, polyolefins and modified corn starch. Naturally, of course, the antiperspirant composition will also ideally include a fragrance.

The foregoing list of materials is by way of example only and is not intended to be a comprehensive list of all potential materials that may be useful in an antiperspirant composition. Obviously, the skilled worker may select materials which provide the desired application and aesthetic characteristics of the particular form of antiperspirant composition to be produced.

The topical antiperspirant composition of the present invention may be formulated as a lotion, cream, gel, soft-solid, solid stick, etc. A cream, soft-solid or solid stick is preferred, with a solid stick being more preferred.

The present invention also embraces a method of inhibiting or reducing perspiration by topically applying an effective amount of an anhydrous antiperspirant composition as described herein to the skin of a human, preferably to the axilla, where such reduction in perspiration is desired. An effective amount is that amount which provides at least a 20% sweat reduction, preferably at least a 40% sweat reduction, when tested in accordance with a standard hot room thermal efficacy protocol, and most preferably that amount which reduces perspiration to a degree that is noticeable by the user. Typically, the amount of antiperspirant composition applied will range from about 0.1 gram to about 1.0 gram per axilla depending on the formulation or such amount as will deliver about 0.01 to about 0.25 gram of antiperspirant active per axilla.

The present invention may be further illustrated by the following example in which the parts and percentages are by weight. EAZCH, when used in the example, means an enhanced efficacy aluminum-zirconium chloro-hydrate-glycine having an HPLC peak 4 to peak 3 area ratio greater than 0.5.

### EXAMPLE

### Solid Stick Antiperspirant

A solid stick antiperspirant composition is prepared having the ingredients and the amounts set out below. The composition is prepared by mixing all of the ingredients (except the fragrance) with the cyclomethicone, heating the mixture to melt the gelling agents, and cooling the mixture to form a solid stick, with the fragrance being added during the cooling step and prior to solidification.

| Ingredient | Weight Percent |
|---|---|
| Cyclomethicone (DC 245) | 27.5 |
| EAZCH | 21.8* |
| Stearyl alcohol | 24.0 |
| C₁₂₋₁₅ alkyl benzoate | 18.0 |
| Hydrogenated castor oil | 2.8 |
| Behenoxy dimethicone | 2.0 |
| C₂₀₋₄₀ Pareth-10 | 1.0 |
| Silica | 0.8 |
| Fragrance/extracts | 2.1 |

| | |
|---|---|
| * Approximately 18% USP active | |

The above composition has good antiperspirant efficacy with excellent skin feel, moisturization and softer, less noticeable hair (or hair stubble).

## Claims

1. An anhydrous topical antiperspirant composition comprising a dermatologically acceptable anhydrous carrier, a perspiration reducing effective amount of a particulate antiperspirant salt suspended in the carrier, 0.3% to 3% by weight behenoxy dimethicone, 3% to 30% by weight non-polar, non- volatile liquid emollient oil, and 0.5% to 5% by weight surfactant.

2. The antiperspirant composition of claim 1, comprising, by weight, about 3% to 25% (USP) antiperspirant salt.

3. The antiperspirant composition of clam 1, comprising, by weight, about 6% to 22% (USP) antiperspirant salt, about 0.5% to about 2.5% behenoxy dimethicone, and about 5% to about 25% emollient oil.

4. The antiperspirant composition of claim 3, comprising about 1% to about 3% by weight surfactant.

5. The antiperspirant composition of claim 1 or 4, wherein the carrier comprises volatile silicone.

6. The antiperspirant composition of clam 5, wherein the emollient oil comprises a fatty alkyl benzoate.

7. The antiperspirant composition of claim 3, wherein the emollient oil comprises C₁₂₋₁₅ alkyl benzoate.

8. The antiperspirant composition of claim 4, wherein the emollient oil comprises C₁₂₋₁₅ alkyl benzoate.

9. The antiperspirant composition of clam 8, wherein the surfactant comprises C₂₀₋₄₀ Pareth-10.

10. The antiperspirant composition of clam 3, 4 or 9, in the form of a cream, soft-solid, or solid stick.

11. A non-therapeutic method of reducing perspiration from human skin comprising applying to human skin a topical antiperspirant composition according to claim 1, 3, 4 or 8.

## Patentansprüche

1. Wasserfreie topische Antitranspirant-Zusammensetzung, umfassend einen hautverträglichen wasserfreien Träger, eine transpirationsreduzierende wirksame Menge eines teilchenförmigen Antitranspirant-Salzes, das in dem Träger suspendiert ist, 0,3 Gew.-% bis 3 Gew.-% Behenoxydimethicon, 3 Gew.-% bis 30 Gew.-% nichtpolares, nichtflüchtiges flüssiges Weichmacheröl und 0,5 Gew. % bis 5 Gew.-% Tensid.

2. Antitranspirant-Zusammensetzung nach Anspruch 1, umfassend, bezogen auf das Gewicht, etwa 3 % bis 25 % (USP) Antitranspirant-Salz.

3. Antitranspirant-Zusammensetzung nach Anspruch 1, umfassend, bezogen auf das Gewicht, etwa 6 % bis 22 % (USP) Antitranspirant-Salz, etwa 0,5 % bis etwa 2,5 % Behenoxydimethicon und etwa 5 % bis etwa 25 % Weichmacheröl.

4. Antitranspirant-Zusammensetzung nach Anspruch 3, umfassend etwa 1 Gew.-% bis etwa 3 Gew.-% Tensid.

5. Antitranspirant-Zusammensetzung nach Anspruch 1 oder 4, wobei der Träger flüchtiges Silikon umfasst.

6. Antitranspirant-Zusammensetzung nach Anspruch 5, wobei das Weichmacheröl ein Fettalkylbenzoat umfasst.

7. Antitranspirant-Zusammensetzung nach Anspruch 3, wobei das Weichmacheröl C₁₂₋₁₅ -Alkylbenzoat umfasst.

8. Antitranspirant-Zusammensetzung nach Anspruch 4, wobei das Weichmacheröl C₁₂₋₁₅ -Alkylbenzoat umfasst.

9. Antitranspirant-Zusammensetzung nach Anspruch 8, wobei das Tensid C₂₀₋₄₀-Pareth-10 umfasst.

10. Antitranspirant-Zusammensetzung nach Anspruch 3, 4 oder 9 in Form einer Creme, eines weichen Feststoffs oder eines festen Stifts.

11. Nichttherapeutisches Verfahren zur Reduzierung der Transpiration aus menschlicher Haut, umfassend das Auftragen einer topischen Antitranspirant-Zusammensetzung nach Anspruch 1, 3, 4 oder 8 auf menschliche Haut.

## Revendications

1. Composition anti-transpirante topique anhydre comprenant un véhicule anhydre acceptable du point de vue dermatologique, une quantité efficace pour réduire la transpiration d'un sel anti-transpirant particulaire en suspension dans le véhicule, 0,3 % à 3 % en poids de béhénoxy dimethicone, 3 % à 30 % en poids d'une huile émolliente liquide apolaire, non volatile, et 0,5 % à 5 % en poids d'agent tensioactif.

2. Composition anti-transpirante selon la revendication 1 comprenant, en poids, environ 3 % à 25 % (USP) de sel anti-transpirant.

3. Composition anti-transpirante selon la revendication 1, comprenant, en poids, environ 6 % à 22 % (USP) de sel anti-transpirant, environ 0,5 % à environ 2,5 % de béhénoxy dimethicone, et environ 5 % à environ 25 % d'huile émolliente.

4. Composition anti-transpirante selon la revendication 1, comprenant environ 1 % à environ 3 % en poids d'agent tensioactif.

5. Composition anti-transpirante selon la revendication 1 ou 4, dans laquelle le véhicule comprend une silicone volatile.

6. Composition anti-transpirante selon la revendication 5, dans laquelle l'huile émolliente comprend un benzoate d'alkyle gras.

7. Composition anti-transpirante selon la revendication 3, dans laquelle l'huile émolliente comprend un benzoate d'alkyle en C_{12 à 15}.

8. Composition anti-transpirante selon la revendication 4, dans laquelle l'huile émolliente comprend un benzoate d'alkyle en C_{12 à 15}.

9. Composition anti-transpirante selon la revendication 8, dans laquelle l'agent tensioactif comprend du Pareth-10 en C_{20 à 40}.

10. Composition anti-transpirante selon la revendication 3, 4 ou 9, sous la forme d'une crème, d'un solide mou, ou d'un bâton solide.

11. Procédé non thérapeutique de réduction de la transpiration de la peau humaine comprenant l'application sur la peau humaine d'une composition anti-transpirante topique selon la revendication 1, 3, 4 ou 8.
